# FASCICULE DE BREVET EUROPEEN

(11) **EP 0 577 523 B1**
(45) Date de publication et mention de la délivrance du brevet: **22.04.1998**
(21) Numéro de dépôt: 93420061.9
(22) Date de dépôt: 10.02.1993
(51) Int. Cl.: C12Q 1/68

(54) **Fragment de l'ADN génomique de Streptococcus pneumoniae, sonde d'hybridation, amorce d'amplification, réactif et procédé de détection de Streptococcus pneumoniae**
Genomische DNS-Fragmente von Streptococcus Pneumoniae, Hybridationssonden, Amplifikationsprimern und Verfahren für dessen Nachweis
Genomic DNA fragments from Streptococcus pneumoniae, hybridisation probes, amplification primers, reagents and method to detect Streptococcus pneumoniae

(30) Priorité: 10.02.1992 FR 9201655
(43) Date de publication de la demande: 05.01.1994
(73) Titulaire: BIO MERIEUX, 69280 Marcy l'Etoile (FR)
(72) Inventeur: Claverys, Jean-Pierre, F-31400 Toulouse (FR); Mabilat, Claude, F-69100 Villeurbanne (FR)
(74) Mandataire: Guerre, Dominique

(56) Documents cités:
- EP-A- 0 305 145
- WO-A-91/08305
- JOURNAL OF BACTERIOLOGY vol. 172, no. 7, Juillet 1990, BALTIMORE US pages 3669 - 3674 B. A. RADNIS ET AL. 'Genetic transformation in streptococus pneumoniae: nucleotide sequence and predicted amino acid sequence of recP'
- EUROPEAN JOURNAL OF CLINICAL MICROBIOLOGY AND INFECTIOUS DISEASES vol. 9, Juin 1990, WIESBADEN, D pages 396 - 401 A. FENOLL ET AL. 'Identification of atypical strains of streptococus pneumoniae by a specific DNA probe'
- J.Bacteriol.,vol.171,1989,pp.5332-5338,(Prudhome et al.).

## Description

La présente invention concerne un fragment de l'ADN génomique de la bactérie *Streptococcus pneumoniae,* une sonde susceptible de s'hybrider spécifiquement avec l'ADN génomique de *Streptococcus pneumoniae,* une amorce pour amplifier spécifiquement l'ADN génomique de *Streptococcus pneumoniae,* un réactif et un procédé pour détecter sélectivement, dans un échantillon biologique, ladite bactérie, mis en oeuvre avec la sonde de l'invention.

Des recherches ont porté sur l'isolement de deux fragments nucléotidiques, sur leur séquençage, leur spécificité vis-à-vis de l'ADN génomique de *Streptococcus pneumoniae* et leur utilisation pour fabriquer des sondes d'hybridation destinées à un procédé de diagnostic.

Ces deux fragments sont respectivement le gène *hexB,* déposé et accessible à la banque de gènes EMBL (European Molecular Biology Laboratory, Heidelberg, RFA) sous le numéro M29686 et dont l'étude a notamment fait l'objet de la publication de M. PRUDHOMME, B. MARTIN, V. MEJEAN et J.P. CLAVERYS (1989) J. Bacteriol. 171, 5332-5338 et qui code pour une protéine essentielle du système de réparation de mésappariements de l'ADN de *Streptococcus pneumoniae,* et l'opéron ami, déposé et accessible à la banque de gènes EMBL sous le numéro X17337 et dont l'étude a notamment fait l'objet de la publication de G. ALLOING, M.C. TROMBE et J.P. CLAVERYS (1990) Mol. Microbiol., 4, 633-644, et qui est impliqué dans le transport des oligopeptides chez le pneumocoque.

Pour chacun de ces deux fragments, divers sous-fragments ont été préparés et utilisés comme sonde d'hybridation de l'ADN génomique de *Streptococcus pneumoniae* . Les sondes les plus couramment utilisées sont pour le gène *hexB,* le fragment *hexB-S*_{*7*} obtenu par action des enzymes de restriction HindIII-Bgl/II (du nucléotide 1321 au nucléotide 1776), de 455 nucléotides et pour l'opéron *ami,* le fragment *ami-S*_{*2*}, obtenu par action des enzymes de restriction BamHI-EcoRI (du nucléotide 2419 au nucléotide 3564), de 1145 nucléotides.

Chacune de ces deux sondes a été soumise à des expériences d'hybridation selon la technique dite "Dot-Blot" selon MANIATIS et coll. (1982) Molecular Cloning, Cold Spring Harbor, avec l'ADN génomique de *Streptococcus pneumoniae* et l'ADN génomique d'autres genres et espèces de bactéries, en conditions stringentes (50% formamide, à 42°C), sur membranes de Nylon (nom commercial Biodyne A, de la Société Pall), pour deux concentrations respectivement 10ng et 100ng d'ADN génomique.

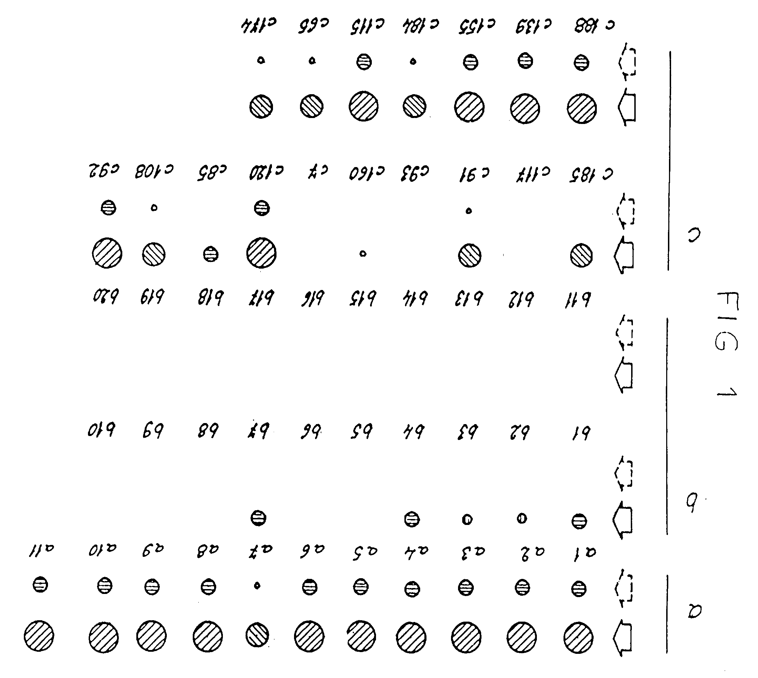

Des résultats identiques ont été obtenus avec les deux sondes *hexB-S*_{*7*} et *ami-S*_{*2*}. La Figure 1 montre les résultats obtenus avec la sonde *ami-S*_{*2*} préalablement marquée par un marquage radioactif avec ³²P (nom commercial Kit Multi-prime de la Société Amersham), les hybridations étant révélées par autoradiographie, pendant 12 heures à -70°C. Selon Figure 1, deux dépôts d'ADN respectivement de 10ng (flèche en pointillé) et de 100ng (flèche en trait plein) ont été réalisés pour chacune des souches bactériennes utilisées. Les souches de bactéries sont regroupées par séries et numérotées au sein de chaque série comme suit :
**Série a**
   a₁-a₁₁ : Isolats clinique de *Streptococcus pneumoniae* appartenant à différents sérotypes.
**Série b :**
   b₁-b₅ *Streptococcus oralis* de la collection API (BioMérieux SA) (références internes API n° 7902025, 7902072, 8305023, 8040010 accessible à la National Culture Type Collection sous la référence NCTC11427, 8408077).
   b₆-b₁₀ : Isolats cliniques classifiés *Streptococcus sanguis* à partir de tests API-20 Strep (BioMérieux SA) dont le résultat est indiqué entre parenthèses ci-après dans l'ordre, SI (4061440), SII (0260451), SII (0270441), SI (0061440), SII (0240440).
   b₁₁-b₁₂ : Isolats cliniques classifiés *Streptococcus mitis* à partir des tests API-20 Strep (0040401 pour les deux souches).
   b₁₃-b₁₄ : Isolats cliniques classifiés *Streptococcus milleri* à partir des tests API-20 Strep (1061010 pour les deux souches).
   b₁₅-b₁₆ : Isolats cliniques classifiés *Streptococcus salivarius* à partir des tests API-20 Strep (5060451 et 5060461).
   b₁₇-b₂₀ : Isolats cliniques d'*Enterococcus faecalis, Listeria monocytogènes, Haemophilus influenzae* et *Neisseria meningitidis.*
**Série c :**
   c : "Streptocoques atypiques" provenant d'isolats cliniques.

Selon Figure 1, les résultats sont les suivants :
- Toutes les souches de *Streptococcus pneumoniae* de la série a) donnent des signaux très visibles qui'sont proportionnels à la concentration considérée.
- Les streptocoques atypiques de la série C, C₉₁, C₁₂₀, C₁₀₈, C₉₂, C₁₈₈, C₁₃₉, C₁₅₅, C₁₈₄, C₁₁₅, C₆₅ et C₁₇₄ donnent les mêmes signaux que ceux de la série a) et pourraient alors être classifiés, selon ce test, dans l'espèce des *Streptococcus pneumoniae*.
- Toutes les souches de *Streptococcus oralis* de la série b), à l'exception de la souche b₅, la souche *Streptococcus mitis* b₁₁ et les streptocoques atypiques de la série C, C₁₈₅, C₁₆₀, C₈₅ donnent un signal visible pour la concentration de 100ng, l'intensité du signal étant environ 10 fois plus faible que celle obtenue pour les *Streptococcus pneumoniae* à la même concentration.

Ces résultats mettent donc en évidence le manque de spécificité des sondes respectivement *hexB-S*_{*7*} et *ami-S*_{*2*} pour *Streptococcus pneumoniae*, au sein du genre *Streptococcus* puisqu'une hybridation partielle, mais néanmoins significative, est détectée avec les espèces *Streptococcus oralis*, espèce la plus voisine de *Streptococcus pneumoniae,* et *Streptococcus mitis*. Ces sondes sont en conséquence non satisfaisantes pour l'obtention d'un test discriminant pour détecter *Streptococcus pneumoniae* parmi d'autres espèces bactériennes les plus proches.

Conformément à la publication de A. FENOLL, J.V. MARTINEZ-SUAREZ, R. MUNOZ, J. CASAL et J.L. GARCIA, Eur. J. Clin. Microbiol. Infect. Dis., 9 (juin 1990) 396-401, d'autres recherches ont conduit à l'élaboration d'une sonde (pCE3) d'hybridation de l'ADN génômique de *Streptococcus pneumoniae*, qui est un fragment de 650 paires de bases, isolé à partir du gène *lyt A*, ce dernier codant pour l'extrémité N-terminale de l'autolysine Streptococcique, l'amidase.

Cette sonde a été testée sur 44 souches de Streptocoques parmi lesquelles 27 étaient identifiées comme des souches de Streptocoques atypiques et les 17 autres comme des souches de *Streptococcus viridans*, à partir de tests de caractérisation classiques.

Bien que cette sonde apporte une solution au problème de l'identification des *Streptococcus pneumoniae* et en particulier parmi les Streptocoques atypiques, elle comporte néanmoins deux inconvénients :
- la sonde utilisée est un fragment de 650 paires de bases et sa production industrielle n'est en conséquence pas aisée,
- et surtout, la spécificité de cette sonde est exclusivement liée à,la présence du gène *lyt A*, dont la copie est unique dans l'ADN génomique de *Streptococcus pneumoniae*, donc elle ne pourra pas détecter ou identifier une souche de *S. pneumoniae* dont le gène *lyt A* a été délété; en outre, ce nombre réduit de copies est désavantageux pour la détection par hybridation directe et pour l'amplification de l'ADN cible.

La présente invention vise à résoudre les problèmes précités de détection sélective de *Streptococcus pneumoniae,* notamment rencontrés en bactériologie médicale.

Le premier objet de l'invention est un fragment monocaténaire de l'ADN génomique de *Streptococcus pneumoniae* comprenant au moins une séquence nucléotidique présentant au moins 70% d'homologie avec au moins une séquence nucléotidique choisie parmi les séquences nucléotidiques SEQ ID NO 2, SEQ ID NO 3, SEQ ID NO 4, représentées en fin de description, et leurs séquences complémentaires respectives. Par séquence complémentaire, on entend toute séquence s'hybridant totalement avec la séquence représentée.

De préférence, la séquence nucléotidique du fragment selon l'invention présente au moins 85% d'homologie avec au moins une desdites séquences nucléotidiques.

Un second objet de l'invention met directement en application ledit fragment et consiste en une sonde susceptible de s'hybrider spécifiquement avec l'ADN génomique de *Streptococcus pneumoniae,* ladite sonde comprenant une séquence nucléotidique présentant au moins 70% d'homologie avec au moins une partie d'une séquence consensus de l'ADN génomique de *Streptococcus pneumoniae,* cette séquence consensus étant choisie parmi les séquences nucléotidiques SEQ ID NO 2, SEQ ID NO 3, SEQ ID NO 4 et leurs séquences complémentaires respectives.

De préférence, la séquence nucléotidique de la sonde de l'invention présente au moins 85% d'homologie avec au moins une partie de ladite séquence consensus.

La sonde de l'invention comprend avantageusement au moins 12 nucléotides.

Préférentiellement, la sonde de l'invention comprend la séquence nucléotidique, SEQ ID NO 3.

Quand elle comprend la SEQ ID NO 3, elle peut être flanquée à son extrémité 5' de la séquence nucléotidique SEQ ID NO 2 et/ou à son extrémité 3' de la séquence nucléotidique SEQ ID NO 4.

La séquence nucléotidique, SEQ ID NO 3, peut être répétée et elle l'est avantageusement quatre fois de manière contiguë.

Selon l'invention, une sonde peut présenter une séquence nucléotidique plus courte et être choisie parmi les séquences SEQ ID NO 5, SEQ ID NO 6 et SEQ ID NO 7, présentées en fin de description.

Le marquage de la sonde n'influe pas sur sa spécificité vis-à-vis de l'ADN génomique de *Streptococcus pneumoniae* et un marqueur approprié est de préférence choisi parmi des isotopes radioactifs, des enzymes choisis parmi la péroxydase de raifort et la phosphatase alcaline et ceux susceptibles d'hydrolyser un substrat chromogène, fluorigène ou luminescent, des composés chimiques chromophores, des composés chromogènes, fluorigènes ou luminescents, des analogues de bases nucléotidiques et la biotine.

A des fins d'utilisation *in vivo* d'une sonde de l'invention, la structure moléculaire de cette dernière est chimiquement modifiée. Des modifications chimiques appropriées, qui permettent d'améliorer la résistance vis-à-vis d'une dégradation enzymatique, en particulier due aux nucléases, et d'accroître en outre le rendement d'hybridation, n'affecte bien entendu pas l'enchaînement des bases. Des exemples en sont l'introduction entre au moins deux nucléotides d'un groupement choisi parmi les esters de diphosphate, d'alkyl- et acryl-phosphonate et de phosphorothioate ou le remplacement d'au moins un désoxyribose par un polyamide.

Un troisième objet de l'invention est une amorce (ou "primer") pour la polymérisation spécifique de l'ADN génomique de *Streptococcus pneumoniae*, en vue de réaliser une amplification de ce dernier. Cette amorce comprend une séquence nucléotidique présentant au moins 70% d'homologie avec au moins une partie d'une séquence consensus de l'ADN génomique de *Streptococcus pneumoniae*, cette séquence consensus étant choisie parmi les séquences nucléotidiques SEQ ID NO 2, SEQ ID NO 3, SEQ ID NO 4 et leurs séquences complémentaires respectives.

De préférence, la séquence nucléotidique d'une amorce de l'invention est choisie parmi les séquences SEQ ID NO 8 à SEQ ID NO 21, représentées en fin de description.

Selon les techniques d'amplification envisagées, il est préférable d'utiliser un couple d'amorces comprenant au moins une amorce de l'invention.

Dans le cas où le couple est constitué par deux amorces de l'invention, ledit couple est avantageusement choisi parmi les couples de sonde constitués par une sonde répondant à la séquence nucléotidique SEQ ID NO 8 et une sonde répondant à l'une quelconque des séquences nucléotidiques SEQ ID NO 11, SEQ ID NO 13, SEQ ID NO 15, SEQ ID NO 17, SEQ ID NO 19 et SEQ ID NO 21 et parmi les couples de sonde constitués par une sonde répondant à la séquence nucléotidique SEQ ID NO 10 et une sonde répondant à l'une quelconque des séquences nucléotidiques SEQ ID NO 13, SEQ ID NO 15, SEQ ID NO 19 et SEQ ID NO 21.

Un quatrième objet de l'invention est un réactif pour détecter sélectivement Streptococcus pneumoniae dans un échantillon biologique, mettant en application une sonde de l'invention telle que décrite ci-dessus.

Si la technique d'hybridation prévue est la technique dite "sandwich", le réactif de l'invention comprend une sonde de capture et une sonde de détection, présentant les caractéristiques d'une sonde de l'invention, la sonde de détection étant notamment marquée au moyen d'un des marqueurs décrits précédemment.

On appelle sonde de capture tout polynucléotide fixé sur un support macromoléculaire capable de s'hybrider avec une partie (ou zone de capture) d'un acide nucléique à détecter dans un échantillon (la cible), en particulier de l'ADN. La sonde de capture peut être un fragment d'acide nucléique naturel (en particulier de l'ADN), un oligonucléotide naturel ou de synthèse ou un fragment d'acide nucléique de synthèse (en particulier de l'ADN) non modifié ou comprenant une ou plusieurs bases modifiées telles que l'inosine, la méthyl-5-désoxycytidine, la désoxyuridine, la diméthylamino-5-désoxyuridine, la diamino-2,6-purine, la bromo-5-désoxyuridine ou toute autre base modifiée permettant l'hybridation. Des modifications chimiques appropriées, qui permettent d'améliorer la résistance vis-à-vis d'une dégradation enzymatique et accroître le rendement d'hybridation peuvent également être envisagées, telles que par exemple l'introduction, entre au moins deux nucléotides, d'un groupement choisi parmi les esters de diphosphate, d'alkyl- et d'acryl-phosphonate et de phosphorothioate ou le remplacement d'au moins un désoxyribose par un polyamide.

La sonde de détection est une sonde capable de s'hybrider à une partie de la cible (zone de détection) qui répond à la définition ci-dessus et est marquée par tout marqueur approprié et notamment choisi parmi les marqueurs cités précédemment.

Dans le réactif, la sonde de l'invention est en milieu liquide fixée sur un support solide, directement ou indirectement. Ledit support est sous toute forme appropriée telle que tube, cône, puits, plaque de microtitration, feuille, polymère soluble. Il est constitué par un matériau naturel, de synthèse, modifié chimiquement ou non et est, selon la technique retenue, choisi parmi les polystyrènes, les copolymères styrènebutadiène, les copolymères styrène-butadiène en mélange avec des polystyrènes, des polypropylènes, des polycarbonates, des copolymères polystyrène-acrylonitrile, des copolymères styrène-méthylméthacrylate de méthyle, parmi les fibres synthétiques Nylon et naturelles, parmi les polysaccharides et les dérivés de la cellulose.

En outre, le réactif de l'invention peut contenir au moins une amorce telle que décrite ci-dessus, pour permettre de mettre en oeuvre une technique d'amplification avant la détection sélective de *Streptococcus pneumoniae,* et de préférence il contient un couple d'amorces selon l'invention.

Le dernier objet de la présente invention est un procédé de détection sélective de *Streptococcus pneumoniae* dans un échantillon biologique, consistant à exposer l'ADN génomique des bactéries contenues dans ledit échantillon, sous forme de fragments monocaténaires, à une sonde de l'invention, puis à détecter les zones d'hybridation avec ladite sonde.

La plupart des techniques d'hybridation connues peuvent être mises en oeuvre dans ce procédé et notamment les techniques dites "Dot Blot", "Southern" et d'hybridation "sandwich". Pour la mise en oeuvre de cette dernière technique, le procédé consiste à exposer préalablement l'ADN génomique des bactéries de l'échantillon à une sonde de capture de l'invention, sur laquelle se fixera spécifiquement l'ADN génomique du *Streptococcus pneumoniae,* puis à exposer l'ADN fixé à une sonde de détection de l'invention.

Selon l'invention, le procédé comprend avantageusement une étape d'amplification de l'ADN génomique de *Streptococcus pneumonia,* en présence d'un système enzymatique adapté et au moins une amorce et notamment un couple d'amorces de l'invention, préalablement à l'étape de détection de *Streptococcus pneumoniae.*

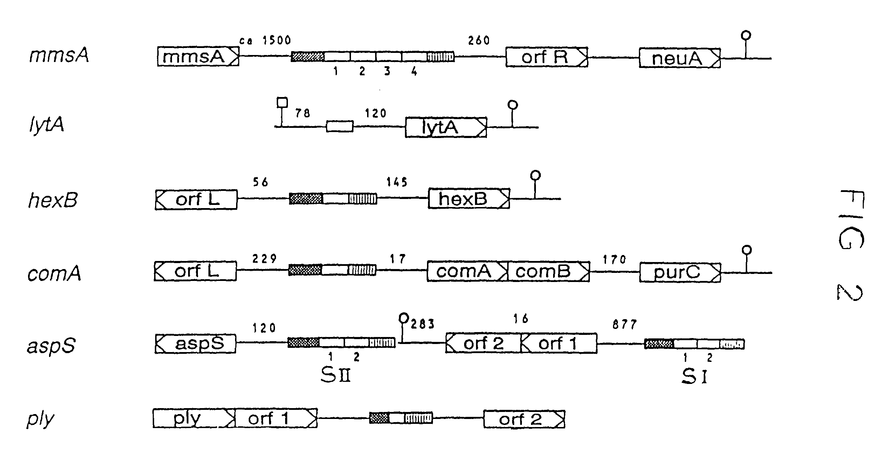

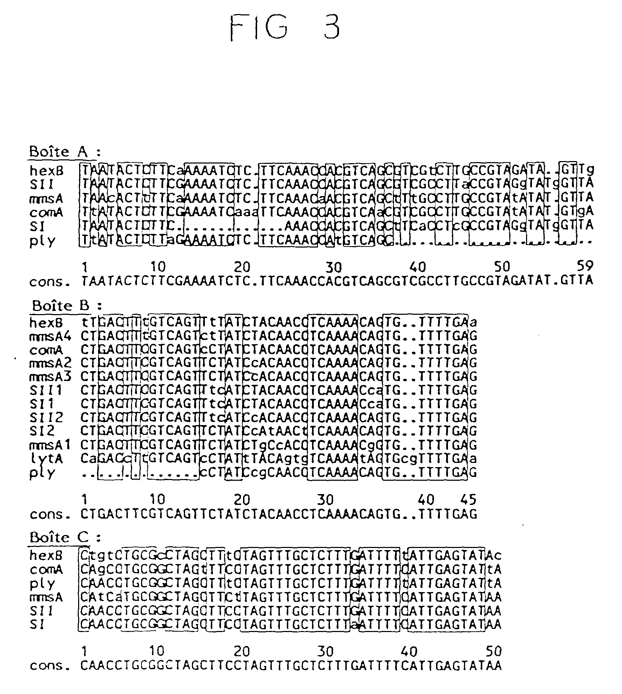

La génèse de l'invention et son intérêt sont à présent exposés selon les étapes 1 à 4 et à l'appui des figures 2 et 3 selon lesquelles :
- figure 2, permet de localiser, au voisinage de différents gènes de *Streptococcus pneumoniae,* les sénucléotidiques répétées appelées respectivement *boiteA,* boîteB, boîteC
- figure 3 illustre la détermination des séquences consensus respectivement SEQ ID NO 2, SEQ ID NO 3 et SEQ ID NO 4 par alignement des différentes séquences répétées au voisinage de 5 gènes de *Streptococcus pneumoniae*.

### ETAPE 1 - Isolement d'une séquence répétée dans l'ADN génomique de Streptococcus pneumoniae

L'isolement par les techniques de recombinaison génétique *in vitro* au cours des travaux développés sur l'étude de *Streptococcus pneumoniae* d'une séquence d'ADN génomique, a permis de mettre en évidence un fragment présentant une homologie, à l'aide de la technique d'hybridation avec de l'ADN total selon la technique SOUTHERN avec plusieurs autres fragments génomiques. Cette situation se traduit par l'apparition de 20 à 25 fragments. Ce fragment s'est montré expérimentalement spécifique de *Streptococcus pneumoniae*. La détermination de sa séquence a permis de mettre en évidence un gène nouveau désigné *mmsA* pouvant être impliqué dans les mécanismes moléculaires de réparation et de recombinaison de l'ADN.

Une séquence particulière responsable de ces hybridations multiples a été localisée sur ce fragment. Il s'agit d'une séquence nucléotidique située dans la région aval, dans le sens 5'--->3', du gène *mmsA*. Cette séquence obtenue par action des enzymes de restriction HpaI et PvuII, compte 340 paires de bases, selon la SEQ ID NO 1 donnée en fin de description. La séquence nucléotidique complète des deux brins complémentaires de ce fragment a été déterminée par la méthode de termination de chaîne (selon SANGER et coll., Proc. Natl. Acad. Sci. USA, 1977, 74, 5463-5467) à partir de matrices d'ADN simple brin du phage M13. On a alors mis en évidence, à l'intérieur de cette séquence, l'existence d'une séquence de 45 nucléotides appelée *boîteB*, directement répétée 4 fois.

Cette séquence *boîteB* a ensuite été retrouvée par comparaison de séquences dans des régions en amont des gènes *hexB, comA, lytA, ply, SI, SII*. On a constaté que ces copies de *boîteB* pouvaient être flanquées en 5' et en 3' de séquences d'une cinquantaine de nucléotides, elles aussi conservées, appelées respectivement *boîteA* et *boîteC*. Pour chacune de ces boîtes, respectivement A, B, C, on a déterminé par alignement des différentes séquences nucléotidiques des régions de *hexB, comA, lytA, ply, SI, SII*, une séquence consensus correspondant à l'enchaînement nucléotidique le plus fréquemment retrouvé dans ces régions. Les séquences consensus SEQ ID NO 2, SEQ ID NO 3 et SEQ ID NO 4 correspondent aux *boîtes* respectivement *A,B, C*.

### Organisation et localisation chromosomique :

L'organisation générale de ces régions répétées dans l'ADN génomique de *Streptococcus pneumoniae* est schématisée sur la figure 2.

Les sites chromosomiques incluant les séquences situées au voisinage des gènes *hexB, comA, lytA et mmsA* ont été caractérisés. Ces sites sont localisés à des endroits différents sur la carte chromosomique de*Streptococcus pneumoniae* établie par séparation de fragments d'ADN par électrophorèse en champ pulsé (Gasc et coll., 1991). En adoptant une représentation circulaire de cette carte, basée sur une division arbitraire en 60 minutes avec une position 0/60 située en haut du cercle et un sens de rotation horaire, la localisation, exprimée en minutes, des divers fragments est, pour la souche testée :
- *comA* : 7'
- *hexB* :10-11'
- *lytA* : 21-24'
- *mmsA :* 24-26'

Cette observation suggère que les fragments contenant ces régions répétées ont des localisations chromosomiques complètement différentes.

Cette situation est très intéressante car elle garantit que, même en cas de remaniement chromosomique important, nombre de copies de cette séquence répétée seront conservées dans l'ADN génomique.

### ETAPE 2 - Mise au point des sondes de l'invention

Par traitement informatique, on a réalisé les alignements de séquence entre les différentes copies identifiées. Ces alignements ont permis d'obtenir les séquences consensus décrites précédemment pour les copies de *boîteA, boîteB et boîteC*. Ces séquences consensus sont données en fin de description selon les références respectivement SEQ ID NO 2, SEQ ID NO 3 et SEQ ID NO 4.

Selon figure 3, ces alignements ont permis de définir la séquence et la localisation de trois oligodésoxyribonucléotides indiqués en fin de description selon les références respectivement SEQ ID NO 5, SEQ ID NO 6 et SEQ ID NO 7. L'un des ces oligonucléotides est dérivé de l'alignement des copies de *boiteA* (SEQ ID NO 5). Le second oligodésoxyribonucléotide (SEQ ID NO 6) est dérivé de l'alignement des copies de *boîteB,* un troisième (SEQ ID NO 7) est dérivé de l'alignement des copies de *boîteC*. Ces oligodésoxyribonucléotides ainsi que toute autre séquence consensus établie d'après les données de *boîteA, boîteB* et *boîteC* ou toute séquence présentant au moins 70% d'homologie avec l'une des séquences consensus peuvent être utilisés comme sonde spécifique de l'ADN génomique de *Streptococcus pneumoniae.*

### ETAPE 3 - Détermination de la spécificité par hybridation moléculaire à l'aide des sondes SEQ ID NO 6, et SEQ ID NO 7

### a) Choix des souches bactériennes :

La classification de souches bactériennes utilisées est précisée ci-après :
1- Souche de laboratoire R800 de *Streptococcus pneumoniae* (Lefèvre, J.C., Claverys, J.P., et Sicard, A.M. (1979) J. Bacteriol. 138, 80-86), dérivée de la souche R36A (Tiraby, G., Fox, M.S., et Bernheimer, H. (1975) J. Bacteriol. 121, 608-618).
2- Isolat clinique atypique (101/87), non capsulé, résistant à l'optochine, résistant à la lyse par le DOC, mais *lytA*⁺.
3- Souche de *Escherichia coli* GM99 (Prère, M.-F. et Fayet, O. (1986) Microbiol. Lett. 33, 37-41).
4- Isolat clinique classifié *Streptococcus sanguis* II (API-20 Strep 0260451), résistant à l'optochine, pneumolysine négatif.
5- Isolat clinique classifié *Streptococcus sanguis* II (API-20 Strep 0270441), résistance intermédiaire à l'optochine, pneumolysine négatif.
6- Souche OB11 de *Streptococcus gordonii* (ex *Streptococcus sanguis* Challis (Haisman , R.J. et Jenkinson, H.F. 1991. Mutants of *Streptococcus gordonii* Challis over-producing glucosyltransferase. J. Gen. Microbiol. 137, 483-489), biovar 2 d'après Kilian et coll. (Kilian et coll., 1989).
7- Isolat clinique classifié *Streptococcus mitis* (API-20 Strep 0040401).
8- Isolat clinique classifié *Streptococcus mitis* (API-20 Strep 0040401).
9- Souche de *Streptococcus oralis* collection API SYSTEM (réf n°7902072).
10- Souche de *Streptococcus oralis* NCTC 11427 (Ronda et coll., 1988).
11- Isolat clinique *Streptococcus* pneumoniae (sérotype 18).
12- Isolat clinique *Streptococcus* pneumoniae (sérotype 6).
13- Isolat clinique de *Streptococcus pneumoniae* (sérotype 23).

Parmi les espèces de Streptocoques, les résultats d'hybridation ADN-ADN (Kilpper-Bälz, R., Wenzig, P., et Schleifer, K.H. 1985. Molecular relationships and classsification of some viridans streptococci as *Streptococcus oralis* and amended description of *Streptococcus oralis* (Bridge and Sneath 1982) Int. J. Sys. Bact. 35, 482-488) montrent que *Streptococcus oralis* qui comprend diverses souches classifiées antérieurement *S. sanguis II, S. mitior, S. viridans, et S. mitis* sont les deux espèces les plus proches de *Streptococcus pneumoniae*. La souche de *Streptococcus oralis* NCTC 11427 retenue dans cette étude est la souche type (Kilpper-Bälz et coll., 1985, et Coykendall, A.L. 1989, Classification and identification of Viridans Streptococci. Clin. Microbiol. Rev. 2, 315-328, Kilian, M., Mikkelsen, L., et Henrichsen, H. 1989, Taxonomic study of Viridans Strepotococci: description of *Streptococcus gordonii* sp. nov. and amended descriptions of *Streptococcus sanguis* (White and Niven 1946), *Streptococcus oralis* (Bridge and Sneath 1982); and *Streptococcus mitis* (Andrewes and Horder, 1906), Int. J. Sys. Bact. 39, 471-484). C'est d'ailleurs à partir de cette souche qu'a été isolé le fragment d'ADN qui constitue une sonde spécifique pour *Streptococcus oralis* (Schmidhuber et coll., 1988). *Streptococcus mitis* est représenté par deux isolats cliniques.

*Streptococcus gordonii*, espèce nouvellement créée (Kilian et coll., 1989) qui regroupe nombre de souches classifiées antérieurement *S. sanguis* II, représentée par la souche OB11 (ex *S. sanguis* Challis) (Kilian et coll., 1989, Haisman et Jenkinson, 1991), ainsi que *S. sanguis* qui n'est pas représenté dans cette étude peuvent être considérés, d'après les résultats d'hybridation ADN-ADN, comme moins proches de *Streptococcus pneumoniae* que *Streptococcus oralis Streptococcus mitis*.

Les résultats des expériences d'hybridation ADN-ADN de Kilpper-Bälz et coll. (1985) dans lesquelles figurent au moins une souche typique de chacune de ces espèces sont résumés dans la figure 8.

### b) Préparation des échantillons, électrophorèse sur gel et transfert sur membrane nylon :

L'ADN chromosomique des différentes souches de streptocoques a été préparé par la technique décrite par Fenoll et coll. (1990). La digestion enzymatique par l'enzyme *Pstl* ainsi que les électrophorèses sur gel d'agarose et les transferts sur membrane Nylon chargée (Biodyne B, provenance PALL) ont été effectuées dans les conditions décrites par Maniatis, T., Fristsch, E.F., et Sambrook, (1982). Molecular cloning ; A laboratory manual (Cold Spring Harbor, NY: Cold Spring Harbor Laboratory).

### c) Conditions d'hybridation moléculaire :

L'oligodésoxyribonucléotide a été marqué en 5' par l'ADN kinase du bactériophage T4 (provenance Bethesda Research Laboratory) avec de l'ATP γ³²P(à 3000 Ci/mM). 50 µl de solution d'oligonucléotide marqué (1x10⁷ cpm pour environ 2,5 picomoles) ont été introduits dans un tampon d'hybridation [6xSSC (Saline Sodium Citrate), 10x Denhardt's, 0,1% SDS (dodécyl sulfate de Na)], 50 mg/ml (ADN de sperme de saumon, 1% de réactif bloquant boehringer) (Maniatis et coll., 1982). L'hybridation a été réalisée à 40°C (sonde de SEQ ID NO 6) ou 48°C (sonde de SEQ ID NO 7), pendant environ 15 heures. Après deux lavages brefs dans une solution de 6xSSC, 0,1% SDS, à température ambiante, la membrane a été mise au contact d'un film radiographique qui a été exposé pendant 3 à 36 heures à -70°C.

Une expérience d'identification de *Streptococcus pneumoniae* par hybridation moléculaire a été réalisée en utilisant comme sonde les oligonucléotides (SEQ ID NO 6 et SEQ ID NO 7). Des ADN chromosomiques de *Streptococcus pneumoniae* et d'autres streptocoques parmi lesquels *Streptococcus oralis* et *Streptococcus gordonii*, deux des espèces les plus proches de cette bactérie ont été digérés par l'enzyme de restriction *Pstl*, séparés par électrophorèse sur gel d'agarose, puis transférés sur membrane Nylon. L'oligonucléotide marqué au ³²P a été mis au contact de cette membrane, en conditions standard d'hybridation.

Les résultats de l'hybridation montrent des signaux d'hybridation très importants obtenus avec l'ADN de *Streptococcus pneumoniae*, alors qu'ils sont inexistants avec l'ADN de *Streptococcus oralis*, espèce la plus proche de *Streptococcus pneumoniae*, ainsi qu'avec l'ADN de *Streptococcus gordonii*, des isolats cliniques classifiés *Streptococcus sanguis*, et d'un des isolats cliniques classifié *Streptococcus mitis*.

### ETAPE 4 - Identification de Streptococcus pneumoniae par hybridation directe sur colonies en utilisant un système de détection non-radioactif et semi-automatisé décrit dans le brevet français FR2663040.

L'identification des souches de *Streptococcus pneumoniae* à partir des souches décrites dans l'étape 3 a été confirmée selon cette technologie de détection non-radioactive.

L'extraction de l'ADN total à partir des colonies est réalisée de la manière suivante. Une colonie bactérienne standardisée à un inoculum 10⁹ bactéries est reprise dans 400µl d'une solution de citrate de sodium 0,1M contenant 0,85g de chlorure de sodium. On ajoute 40 µl de détergent désoxycholate de sodium (1%). Après incubation 5 minutes à température ambiante, 4 extractions phénolchloroforme sont effectuées (Maniatis et coll. 1982). L'ADN est précipité à l'éthanol. Le culot est repris dans 100 µl de tampon citrate de sodium. cette solution est soniquée avec un sonicateur 60W (Société Bioblock sous la réf. C72442) utilisant une sonde de type "cuphorn" (Société Bioblock sous la réf. C72438) afin d'obtenir une population de fragments de taille majoritaire de 1 Kilobase

Un aliquote correspondant à 10⁸ bactéries dans 10 µl est alors identifié par hybridation selon le protocole suivant. Dans une plaque de microtitration (Nom commercial Nunc 439454) est déposée une solution de la sonde oligonucléotidique de capture (sonde SEQ ID NO 6) à 1 ng/µl dans du PBS 1X (0.15 M NaCl, 0.05 M phosphate de sodium, pH 7.0). La plaque est incubée 2 h à 37°C puis lavée 3 fois avec 300 µl de PBST (PSB + détergent de marque TWEEN de la Société MERCK). La cible constituée par 10 µl de l'ADN total soniqué est mélangée avec 70 *µ*l de tampon PBS saumon [PBS3X + ADN de sperme de saumon 10 *µ*l/ml, (Société Sigma sous la réf. D9156)] et 10 µl de soude 2N. L'ensemble est neutralisé 5 minutes après, par l'addition de 10 µl d'acide acétique 2N. L'ensemble est ajouté dans le puits en plus de 50 µl d'une solution du conjugé sonde de détection marquée à la péroxydase selon SEQ ID NO 7 à la concentration de 0,1 ng/*µ*l dans un tampon PBS cheval [PBS3X + 10% sérum de cheval, (Société bioMérieux SA réf. 55842)].

La plaque est incubée 1 h à 37°C et lavée par 3X 300 *µ*l de PBS Tween [PBS1X + 0.5% Tween 20 (Société Merck réf 822184)].

100 *µ*l de substrat OPD (ortho-phenylenediamine de Cambridge Medical Biotechnology ref/456) dans un tampon spécifique (0,055 M acide citrique, 0.1 M Na₂HPO₄, pH 4,93) à la concentration de 4 mg/ml auquel on ajoute extemporanément H₂O₂ à 30 volumes au 1/1000, sont ajoutés par puits. Après 20 minutes de réaction, l'activité enzymatique est bloquée par 100 *µ*l d'H₂SO₄ 1N et la lecture est effectuée sur un lecteur de microplaques de la marque déposée Axia Microreader (Société bioMérieux SA) à 492 nm.

Le système ne génère pas de bruit de fond puisque le puits contenant l'ADN de saumon du tampon d'hybridation qui est soniqué de la même manière que l'ADN des souches testées, ne génère pas de signal. Les résultats de spécificité sont les mêmes que que ceux obtenus dans l'étape 3. Cette application d'une sonde froide indique que la spécificité de la séquence de l'invention est conservée quelle que soit la méthodologie d'hybridation utilisée.
(1) INFORMATIONS GENERALES:
   (i) DEPOSANT: BIO MERIEUX
   (ii) TITRE DE L'INVENTION: Fragment de l'ADN génomique de Streptococcus pneumoniae, sonde d'hybridation, amorce d'amplification, réactif et procédé de détection de Streptococcus pneumoniae.
   (iii) NOMBRE DE SEQUENCES: 21
   (iv) ADRESSE DE CORRESPONDANCE:
      (A) NOM: Cabinet GERMAIN & MAUREAU
      (B) RUE: 20 Bd Eugène Deruelle
      (C) VILLE: LYON
      (D) PAYS: FRANCE
      (E) CODE POSTAL: 69003
      (F) TELEPHONE: 78 60 24 93
      (G) TELEFAX: 78 60 92 85
   (v) FORME DECHIFFRABLE PAR ORDINATEUR:
      (A) TYPE DE SUPPORT: disquette 3,5 pouces DS, HD
      (B) ORDINATEUR: EPSON (compatible IBM)
      (C) SYSTEME D'EXPLOITATION: DOS
      (D) LOGICIEL: MICROSOFT WORD POUR WINDOWS
   (vi) DEMANDE ACTUELLE:
      (A) DATE DE LA DEMANDE: 10.02.1993
      (B) NUMERO DE LA DEMANDE: 93420061.9
   (vii) DATE DE LA DEMANDE ANTERIEURE: 10.02.92
      (A) NUMERO DE LA DEMANDE: 92 01655
      (B) CLASSIFICATION: C12N, C12Q, C12R
   (viii) MANDATAIRE:
      (A) NOM: CABINET GERMAIN & MAUREAU, Dominique GUERRE
      (B) REFERENCE DOSSIER: MD/B05B1453 EP
2) INFORMATIONS POUR LA SEQ ID NO:1:
   i) CARACTERISTIQUES DE LA SEQUENCE:
      A) LONGUEUR: 340 paires de bases
      B) TYPE: acide nucléique
      C) NOMBRE DE BRINS: simple
      D) CONFIGURATION: linéaire
   ii) TYPE DE MOLECULE: ADN (génomique)
   iii) HYPOTHETIQUE: non
   iv) ANTI-SENS: non
   vi) ORIGINE:
      A) ORGANISME: Streptococcus pneumoniae
      B) SOUCHE: R800 (Souchothèque BIOMERIEUX)
      C) INDIVIDU/ISOLE: R800
   viii) POSITION DANS LE GENOME:
      A) CHROMOSOME/SEGMENT:
      B) POSITION SUR LA CARTE: 24-26 minutes
   ix) CARACTERISTIQUE:
      A) NOM/CLE: unité répétée
      B) EMPLACEMENT: 3..291
      C) METHODE D'IDENTIFICATION: expérimentale
      D) AUTRES RENSEIGNEMENTS: région située sur un fragment de restriction HpaI-PvuII situé environ à 1,5 kilobase en aval du gène mmsA. Dans cette région de 340 paires de base, se trouve une unité répétée de 288 paires de base du nucléotide 3 au nucléotide 291. Cette unité répétée est présente 25 fois dans le génome de Streptococcus pneumoniae.
   xi) DESCRIPTION DE LA SEQUENCE: 
SEQ ID NO 1:
GTTAACACTT TTCAAAAATC TCTTCAAACA ACGTCAGCTT TGCCTTGCCG TATATATGTT 60
ACTGACTTCG TCAGTTCTAT CTGCCACCTC AAAACGGTGT TTTGAGCTGA CTTCGTCAGT 120
TCTATCCACA ACCTCAAAAC AGTGTTTTGA GCTGACTTCG TCAGTTCTAT CCACAACCTC 180
AAAACAGTGT TTTGAGCTGA CTTTGTCAGT CTTATCTACA ACCTCAAAAC AGTGTTTTGA 240
GCATCATGCG GCTAGCTTCT TAGTTTGCTC TTTGATTTTC ATTGAGTATA AAAACAGATG 300
AGTTTCTGTT TTCTTTTTAT GGACTATAAA TGTTCAGCTG 340
3) INFORMATIONS POUR LA SEQ ID NO:2:
   i) CARACTERISTIQUES DE LA SEQUENCE:
      A) LONGUEUR: 59 paires de bases
      B) TYPE: acide nucléique
      C) NOMBRE DE BRINS: simple
      D) CONFIGURATION: linéaire
   ii) TYPE DE MOLECULE: ADN (génomique)
   iii) HYPOTHETIQUE: non
   iv) ANTI-SENS: non
   vi) ORIGINE:
      A) ORGANISME: Streptococcus pneumoniae
      B) SOUCHE: R800 (Souchothèque BIOMERIEUX)
      C) INDIVIDU/ISOLE: R800
   ix) CARACTERISTIQUE:
      A) NOM/CLE: unité répétée
      B) EMPLACEMENT: 1..59
      C) METHODE D'IDENTIFICATION: expérimentale
   xi) DESCRIPTION DE LA SEQUENCE: 
SEQ ID NO 2:
TAATACTCTT CGAAAATCTC TTCAAACCAC GTCAGCGTCG CCTTGCCGTA GATATGTTA
4) INFORMATIONS POUR LA SEQ ID NO:3:
   i) CARACTERISTIQUES DE LA SEQUENCE:
      A) LONGUEUR: 45 paires de bases
      B) TYPE: acide nucléique
      C) NOMBRE DE BRINS: simple
      D) CONFIGURATION: linéaire
   ii) TYPE DE MOLECULE: ADN (génomique)
   iii) HYPOTHETIQUE: non
   iv) ANTI-SENS: non
   vi) ORIGINE:
      A) ORGANISME: Streptococcus pneumoniae
      B) SOUCHE: R800 (Souchothèque BIOMERIEUX)
      C) INDIVIDU/ISOLE: R800
   ix) CARACTERISTIQUE:
      A) NOM/CLE: unité répétée
      B) EMPLACEMENT: 1..45
      C) METHODE D'IDENTIFICATION: expérimentale
   xi) DESCRIPTION DE LA SEQUENCE: 
SEQ ID NO 3:
CTGACTTCGT CAGTTCTATC TACAACCTCA AAACAGTGTT TTGAG
5) INFORMATIONS POUR LA SEQ ID NO:4:
   i) CARACTERISTIQUES DE LA SEQUENCE:
      A) LONGUEUR: 50 paires de bases
      B) TYPE: acide nucléique
      C) NOMBRE DE BRINS: simple
      D) CONFIGURATION: linéaire
   ii) TYPE DE MOLECULE: ADN (génomique)
   iii) HYPOTHETIQUE: non
   iv) ANTI-SENS: non
   vi) ORIGINE:
      A) ORGANISME: Streptococcus pneumoniae
      B) SOUCHE: R800 (Souchothèque BIOMERIEUX)
      C) INDIVIDU/ISOLE: R800
   ix) CARACTERISTIQUE:
      A) NOM/CLE: unité répétée
      B) EMPLACEMENT: 1..50
      C) METHODE D'IDENTIFICATION: expérimentale
   xi) DESCRIPTION DE LA SEQUENCE: 
SEQ ID NO 4:
CAACCTGCGG CTAGCTTCCT AGTTTGCTCT TTGATTTTCA TTGAGTATAA
6) INFORMATIONS POUR LA SEQ ID NO:5:
   i) CARACTERISTIQUES DE LA SEQUENCE:
      A) LONGUEUR: 20 paires de bases
      B) TYPE: acide nucléique
      C) NOMBRE DE BRINS: simple
      D) CONFIGURATION: linéaire
   ii) TYPE DE MOLECULE: ADN (génomique)
   iii) HYPOTHETIQUE: non
   iv) ANTI-SENS: non
   vi) ORIGINE:
      A) ORGANISME: Streptococcus pneumoniae
      B) SOUCHE: R800 (Souchothèque BIOMERIEUX)
      C) INDIVIDU/ISOLE: R800
   ix) CARACTERISTIQUE:
      A) NOM/CLE: unité répétée
      B) EMPLACEMENT: 1..20
      C) METHODE D'IDENTIFICATION: expérimentale
   xi) DESCRIPTION DE LA SEQUENCE: 
SEQ ID NO 5:
ACGTCARCKT YRCCTTRCCG
7) INFORMATIONS POUR LA SEQ ID NO:6:
   i) CARACTERISTIQUES DE LA SEQUENCE:
      A) LONGUEUR: 22 paires de bases
      B) TYPE: acide nucléique
      C) NOMBRE DE BRINS: simple
      D) CONFIGURATION: linéaire
   ii) TYPE DE MOLECULE: ADN (génomique)
   iii) HYPOTHETIQUE: non
   iv) ANTI-SENS: non
   vi) ORIGINE:
      A) ORGANISME: Streptococcus pneumoniae
      B) SOUCHE: R800 (Souchothèque BIOMERIEUX)
      C) INDIVIDU/ISOLE: R800
   ix) CARACTERISTIQUE:
      A) NOM/CLE: unité répétée
      B) EMPLACEMENT: 1..22
      C) METHODE D'IDENTIFICATION: expérimentale
   xi) DESCRIPTION DE LA SEQUENCE: 
SEQ ID NO 6:
TATYYACARY STCAAAAYAG TG
8) INFORMATIONS POUR LA SEQ ID NO:7:
   i) CARACTERISTIQUES DE LA SEQUENCE:
      A) LONGUEUR: 29 paires de bases
      B) TYPE: acide nucléique
      C) NOMBRE DE BRINS: simple
      D) CONFIGURATION: linéaire
   ii) TYPE DE MOLECULE: ADN (génomique)
   iii) HYPOTHETIQUE: non
   iv) ANTI-SENS: non
   vi) ORIGINE:
      A) ORGANISME: Streptococcus pneumoniae
      B) SOUCHE: R800 (Souchothèque BIOMERIEUX)
      C) INDIVIDU/ISOLE: R800
   ix) CARACTERISTIQUE:
      A) NOM/CLE: unité répétée
      B) EMPLACEMENT: 1..29
      C) METHODE D'IDENTIFICATION: expérimentale
   xi) DESCRIPTION DE LA SEQUENCE: 
SEQ ID NO 7:
TAGTTTGCTC TTTGATTTTY ATTGAGTAT
9) INFORMATIONS POUR LA SEQ ID NO:8:
   i) CARACTERISTIQUES DE LA SEQUENCE:
      A) LONGUEUR: 20 paires de bases
      B) TYPE: acide nucléique
      C) NOMBRE DE BRINS: simple
      D) CONFIGURATION: linéaire
   ii) TYPE DE MOLECULE: ADN (génomique)
   iii) HYPOTHETIQUE: non
   iv) ANTI-SENS: non
   vi) ORIGINE:
      A) ORGANISME: Streptococcus pneumoniae
      B) SOUCHE: R800 (Souchothèque BIOMERIEUX)
      C) INDIVIDU/ISOLE: R800
   ix) CARACTERISTIQUE:
      A) NOM/CLE: unité répétée
      B) EMPLACEMENT: 1..20
      C) METHODE D'IDENTIFICATION: expérimentale
   xi) DESCRIPTION DE LA SEQUENCE: 
SEQ ID NO 8:
ACGTCAGCTT TGCCTTGCCG
10) INFORMATIONS POUR LA SEQ ID NO:9:
   i) CARACTERISTIQUES DE LA SEQUENCE:
      A) LONGUEUR: 20 paires de bases
      B) TYPE: acide nucléique
      C) NOMBRE DE BRINS: simple
      D) CONFIGURATION: linéaire
   ii) TYPE DE MOLECULE: ADN (génomique)
   iii) HYPOTHETIQUE: non
   iv) ANTI-SENS: non
   vi) ORIGINE:
      A) ORGANISME: Streptococcus pneumoniae
      B) SOUCHE: R800 (Souchothèque BIOMERIEUX)
      C) INDIVIDU/ISOLE: R800
   ix) CARACTERISTIQUE:
      A) NOM/CLE: unité répétée
      B) EMPLACEMENT: 1..20
      C) METHODE D'IDENTIFICATION: expérimentale
   xi) DESCRIPTION DE LA SEQUENCE: 
SEQ ID NO 9:
CGGCAAGGCA AAGCTGACGT
11) INFORMATIONS POUR LA SEQ ID NO:10:
   i) CARACTERISTIQUES DE LA SEQUENCE:
      A) LONGUEUR: 20 paires de bases
      B) TYPE: acide nucléique
      C) NOMBRE DE BRINS: simple
      D) CONFIGURATION: linéaire
   ii) TYPE DE MOLECULE: ADN (génomique)
   iii) HYPOTHETIQUE: non
   iv) ANTI-SENS: non
   vi) ORIGINE:
      A) ORGANISME: Streptococcus pneumoniae
      B) SOUCHE: R800 (Souchothèque BIOMERIEUX)
      C) INDIVIDU/ISOLE: R800
   ix) CARACTERISTIQUE:
      A) NOM/CLE: unité répétée
      B) EMPLACEMENT: 1..20
      C) METHODE D'IDENTIFICATION: expérimentale
   xi) DESCRIPTION DE LA SEQUENCE: 
SEQ ID NO 10:
ATCTGCCACC TCAAAACGGT
12) INFORMATIONS POUR LA SEQ ID NO:11:
   i) CARACTERISTIQUES DE LA SEQUENCE:
      A) LONGUEUR: 20 paires de bases
      B) TYPE: acide nucléique
      C) NOMBRE DE BRINS: simple
      D) CONFIGURATION: linéaire
   ii) TYPE DE MOLECULE: ADN (génomique)
   iii) HYPOTHETIQUE: non
   iv) ANTI-SENS: non
   vi) ORIGINE:
      A) ORGANISME: Streptococcus pneumoniae
      B) SOUCHE: R800 (Souchothèque BIOMERIEUX)
      C) INDIVIDU/ISOLE: R800
   ix) CARACTERISTIQUE:
      A) NOM/CLE: unité répétée
      B) EMPLACEMENT: 1..20
      C) METHODE D'IDENTIFICATION: expérimentale
   xi) DESCRIPTION DE LA SEQUENCE: 
SEQ ID NO 11:
ACCGTTTTGA GGTGGCAGAT
13) INFORMATIONS POUR LA SEQ ID NO:12:
   i) CARACTERISTIQUES DE LA SEQUENCE:
      A) LONGUEUR: 20 paires de bases
      B) TYPE: acide nucléique
      C) NOMBRE DE BRINS: simple
      D) CONFIGURATION: linéaire
   ii) TYPE DE MOLECULE: ADN (génomique)
   iii) HYPOTHETIQUE: non
   iv) ANTI-SENS: non
   vi) ORIGINE:
      A) ORGANISME: Streptococcus pneumoniae
      B) SOUCHE: R800 (Souchothèque BIOMERIEUX)
      C) INDIVIDU/ISOLE: R800
   ix) CARACTERISTIQUE:
      A) NOM/CLE: unité répétée
      B) EMPLACEMENT: 1..20
      C) METHODE D'IDENTIFICATION: expérimentale
   xi) DESCRIPTION DE LA SEQUENCE: 
SEQ ID NO 12:
ATCCACAACC TCAAAACAGT
14) INFORMATIONS POUR LA SEQ ID NO:13:
   i) CARACTERISTIQUES DE LA SEQUENCE:
      A) LONGUEUR: 20 paires de bases
      B) TYPE: acide nucléique
      C) NOMBRE DE BRINS: simple
      D) CONFIGURATION: linéaire
   ii) TYPE DE MOLECULE: ADN (génomique)
   iii) HYPOTHETIQUE: non
   iv) ANTI-SENS: non
   vi) ORIGINE:
      A) ORGANISME: Streptococcus pneumoniae
      B) SOUCHE: R800 (Souchothèque BIOMERIEUX)
      C) INDIVIDU/ISOLE: R800
   ix) CARACTERISTIQUE:
      A) NOM/CLE: unité répétée
      B) EMPLACEMENT: 1..20
      C) METHODE D'IDENTIFICATION: expérimentale
   xi) DESCRIPTION DE LA SEQUENCE: 
SEQ ID NO 13:
ACTGTTTTGA GGTTGTGGAT
15) INFORMATIONS POUR LA SEQ ID NO:14:
   i) CARACTERISTIQUES DE LA SEQUENCE:
      A) LONGUEUR: 20 paires de bases
      B) TYPE: acide nucléique
      C) NOMBRE DE BRINS: simple
      D) CONFIGURATION: linéaire
   ii) TYPE DE MOLECULE: ADN (génomique)
   iii) HYPOTHETIQUE: non
   iv) ANTI-SENS: non
   vi) ORIGINE:
      A) ORGANISME: Streptococcus pneumoniae
      B) SOUCHE: R800 (Souchothèque BIOMERIEUX)
      C) INDIVIDU/ISOLE: R800
   ix) CARACTERISTIQUE:
      A) NOM/CLE: unité répétée
      B) EMPLACEMENT: 1..20
      C) METHODE D'IDENTIFICATION: expérimentale
   xi) DESCRIPTION DE LA SEQUENCE: 
SEQ ID NO 14:
ATCTACAACC TCAAAACAGT
16) INFORMATIONS POUR LA SEQ ID NO:15:
   i) CARACTERISTIQUES DE LA SEQUENCE:
      A) LONGUEUR: 20 paires de bases
      B) TYPE: acide nucléique
      C) NOMBRE DE BRINS: simple
      D) CONFIGURATION: linéaire
   ii) TYPE DE MOLECULE: ADN (génomique)
   iii) HYPOTHETIQUE: non
   iv) ANTI-SENS: non
   vi) ORIGINE:
      A) ORGANISME: Streptococcus pneumoniae
      B) SOUCHE: R800 (Souchothèque BIOMERIEUX)
      C) INDIVIDU/ISOLE: R800
   ix) CARACTERISTIQUE:
      A) NOM/CLE: unité répétée
      B) EMPLACEMENT: 1..20
      C) METHODE D'IDENTIFICATION: expérimentale
   xi) DESCRIPTION DE LA SEQUENCE: 
SEQ ID NO 15:
ACTGTTTTGA GGTTGTAGAT
17) INFORMATIONS POUR LA SEQ ID NO:16:
   i) CARACTERISTIQUES DE LA SEQUENCE:
      A) LONGUEUR: 20 paires de bases
      B) TYPE: acide nucléique
      C) NOMBRE DE BRINS: simple
      D) CONFIGURATION: linéaire
   ii) TYPE DE MOLECULE: ADN (génomique)
   iii) HYPOTHETIQUE: non
   iv) ANTI-SENS: non
   vi) ORIGINE:
      A) ORGANISME: Streptococcus pneumoniae
      B) SOUCHE: R800 (Souchothèque BIOMERIEUX)
      C) INDIVIDU/ISOLE: R800
   ix) CARACTERISTIQUE:
      A) NOM/CLE: unité répétée
      B) EMPLACEMENT: 1..20
      C) METHODE D'IDENTIFICATION: expérimentale
   xi) DESCRIPTION DE LA SEQUENCE: 
SEQ ID NO 16:
GAGCATCATG CGGCTAGCTT
18) INFORMATIONS POUR LA SEQ ID NO:17:
   i) CARACTERISTIQUES DE LA SEQUENCE:
      A) LONGUEUR: 20 paires de bases
      B) TYPE: acide nucléique
      C) NOMBRE DE BRINS: simple
      D) CONFIGURATION: linéaire
   ii) TYPE DE MOLECULE: ADN (génomique)
   iii) HYPOTHETIQUE: non
   iv) ANTI-SENS: non
   vi) ORIGINE:
      A) ORGANISME: Streptococcus pneumoniae
      B) SOUCHE: R800 (Souchothèque BIOMERIEUX)
      C) INDIVIDU/ISOLE: R800
   ix) CARACTERISTIQUE:
      A) NOM/CLE: unité répétée
      B) EMPLACEMENT: 1..20
      C) METHODE D'IDENTIFICATION: expérimentale
   xi) DESCRIPTION DE LA SEQUENCE: 
SEQ ID NO 17:
AAGCTAGCCG CATGATGCTC
19) INFORMATIONS POUR LA SEQ ID NO:18:
   i) CARACTERISTIQUES DE LA SEQUENCE:
      A) LONGUEUR: 20 paires de bases
      B) TYPE: acide nucléique
      C) NOMBRE DE BRINS: simple
      D) CONFIGURATION: linéaire
   ii) TYPE DE MOLECULE: ADN (génomique)
   iii) HYPOTHETIQUE: non
   iv) ANTI-SENS: non
   vi) ORIGINE:
      A) ORGANISME: Streptococcus pneumoniae
      B) SOUCHE: R800 (Souchothèque BIOMERIEUX)
      C) INDIVIDU/ISOLE: R800
   ix) CARACTERISTIQUE:
      A) NOM/CLE: unité répétée
      B) EMPLACEMENT: 1..20
      C) METHODE D'IDENTIFICATION: expérimentale
   xi) DESCRIPTION DE LA SEQUENCE: 
SEQ ID NO 18:
GCTAGCTTCT TAGTTTGCTC
20) INFORMATIONS POUR LA SEQ ID NO:19:
   i) CARACTERISTIQUES DE LA SEQUENCE:
      A) LONGUEUR: 20 paires de bases
      B) TYPE: acide nucléique
      C) NOMBRE DE BRINS: simple
      D) CONFIGURATION: linéaire
   ii) TYPE DE MOLECULE: ADN (génomique)
   iii) HYPOTHETIQUE: non
   iv) ANTI-SENS: non
   vi) ORIGINE:
      A) ORGANISME: Streptococcus pneumoniae
      B) SOUCHE: R800 (Souchothèque BIOMERIEUX)
      C) INDIVIDU/ISOLE: R800
   ix) CARACTERISTIQUE:
      A) NOM/CLE: unité répétée
      B) EMPLACEMENT: 1..20
      C) METHODE D'IDENTIFICATION: expérimentale
   xi) DESCRIPTION DE LA SEQUENCE: 
SEQ ID NO 19:
GAGCAAACTA AGAAGCTAGC
21) INFORMATIONS POUR LA SEQ ID NO:20:
   i) CARACTERISTIQUES DE LA SEQUENCE:
      A) LONGUEUR: 20 paires de bases
      B) TYPE: acide nucléique
      C) NOMBRE DE BRINS: simple
      D) CONFIGURATION: linéaire
   ii) TYPE DE MOLECULE: ADN (génomique)
   iii) HYPOTHETIQUE: non
   iv) ANTI-SENS: non
   vi) ORIGINE:
      A) ORGANISME: Streptococcus pneumoniae
      B) SOUCHE: R800 (Souchothèque BIOMERIEUX)
      C) INDIVIDU/ISOLE: R800
   ix) CARACTERISTIQUE:
      A) NOM/CLE: unité répétée
      B) EMPLACEMENT: 1..20
      C) METHODE D'IDENTIFICATION: expérimentale
   xi) DESCRIPTION DE LA SEQUENCE: 
SEQ ID NO 20:
TGCTCTTTGA TTTTCATTGA
22) INFORMATIONS POUR LA SEQ ID NO:21:
   i) CARACTERISTIQUES DE LA SEQUENCE:
      A) LONGUEUR: 20 paires de bases
      B) TYPE: acide nucléique
      C) NOMBRE DE BRINS: simple
      D) CONFIGURATION: linéaire
   ii) TYPE DE MOLECULE: ADN (génomique)
   iii) HYPOTHETIQUE: non
   iv) ANTI-SENS: non
   vi) ORIGINE:
      A) ORGANISME: Streptococcus pneumoniae
      B) SOUCHE: R800 (Souchothèque BIOMERIEUX)
      C) INDIVIDU/ISOLE: R800
   ix) CARACTERISTIQUE:
      A) NOM/CLE: unité répétée
      B) EMPLACEMENT: 1..20
      C) METHODE D'IDENTIFICATION: expérimentale
   xi) DESCRIPTION DE LA SEQUENCE:

SEQ ID NO 21:
TCAATGAAAA TCAAAGAGCA

## Revendications

1. Fragment monocaténaire de l'ADN génomique de *Streptococcus pneumoniae,* caractérisé en ce qu'il comprend au moins une séquence nucléotidique présentant au moins 70% d'homologie avec au moins une séquence nucléotidique choisie parmi les séquences nucléotidiques SEQ ID NO 2, SEQ ID NO 3, SEQ ID NO 4 et leurs séquences complémentaires respectives.

2. Fragment selon la revendication 1, caractérisé en ce que la séquence nucléotidique dudit fragment présente au moins 85% d'homologie avec au moins une séquence nucléotidique choisie parmi les séquences nucléotidiques SEQ ID NO 2, SEQ ID NO 3, SEQ ID NO 4 et leurs séquences complémentaires respectives.

3. Sonde susceptible de s'hybrider spécifiquement avec l'ADN génomique de *Streptococcus pneumoniae,* caractérisée en ce qu'elle comprend une séquence nucléotidique qui, à la fois, présente au moins 70% d'homologie avec au moins une partie d'une séquence consensus de l'ADN génomique de *Streptococcus pneumoniae* choisie parmi les séquences nucléotidiques SEQ ID NO 2, SEQ ID NO 3, SEQ ID NO 4 et leurs séquences complémentaires respectives, et s'hybride uniquement avec l'ADN génomique de *Streptococcus pneumoniae.*

4. Sonde selon la revendication 3, caractérisée en ce que la séquence nucléotidique que ladite sonde comprend présente au moins 85% d'homologie avec au moins une partie de ladite séquence consensus.

5. Sonde selon la revendication 3 ou 4, caractérisée en ce que la séquence nucléotidique que ladite sonde comprend, comporte au moins 12 nucléotides.

6. Sonde selon l'une quelconque des revendications 3 à 5, caractérisée en ce qu'elle comprend la séquence nucléotidique, SEQ ID NO 3.

7. Sonde selon la revendication 6, caractérisée en ce que ladite séquence nucléotidique SEQ ID NO 3 est flanquée à son extrémité 5' d'une séquence nucléotidique SEQ ID NO 2.

8. Sonde selon la revendication 6, caractérisée en ce que ladite séquence nucléotidique SEQ ID NO 3 est flanquée à son extrémité 3' d'une séquence nucléotidique SEQ ID NO 4.

9. Sonde selon la revendication 6, caractérisée en ce que la séquence nucléotidique SEQ ID NO 3 est répétée quatre fois de manière contiguë.

10. Sonde selon la revendication 3, caractérisée en ce qu'elle est constituée par une séquence nucléotidique choisie parmi les séquences SEQ ID NO 5, SEQ ID NO 6 et SEQ ID NO 7.

11. Sonde de capture d'ADN dénaturé caractérisée en ce qu'elle est choisie parmi les sondes selon l'une quelconque des revendications 3 à 10.

12. Sonde selon l'une quelconque des revendications 1 à 10, caractérisée en ce qu'elle est marquée au moyen d'un marqueur choisi parmi des isotopes radioactifs, des enzymes choisis parmi la péroxydase et la phosphatase alcaline et ceux susceptibles d'hydrolyser un substrat chromogène, fluorigène ou luminescent, des composés chimiques chromophores, des composés chromogènes, fluorigènes ou luminescents, des analogues de bases nucléotidiques, et la biotine.

13. Sonde de détection caractérisée en ce qu'elle est choisie parmi les sondes selon la revendication 12.

14. Amorce spécifique pour l'amplification par la polymérisation de l'ADN génomique de *Streptococcus pneumoniae,* caractérisée en ce qu'elle comprend une séquence nucléotidique qui, à la fois, présente au moins 70% d'homologie avec au moins une partie d'une séquence consensus de l'ADN génomique de *Streptococcus pneumoniae* choisie parmi les séquences nucléotidiques SEQ ID NO 2, SEQ ID NO 3, SEQ ID NO 4 et leurs séquences complémentaires respectives, et s'hybride uniquement avec l'ADN génomique de *Streptococcus pneumoniae.*

15. Amorce selon la revendication 14, caractérisée en ce qu'elle est choisie parmi les amorces répondant aux séquences nucléotidiques respectivement SEQ ID NO 8 à SEQ ID NO 21.

16. Couple d'amorces caractérisé en ce qu'il comprend au moins une amorce choisie parmi les amorces selon la revendication 15.

17. Couple d'amorces selon la revendication 16, caractérisé en ce qu'il est choisi parmi les couples d'amorces constitués par une amorce répondant à la séquence nucléotidique SEQ ID NO 8 et une sonde répondant à l'une quelconque des séquences nucléotidiques SEQ ID NO 11, SEQ ID NO 13, SEQ ID NO 15, SEQ ID NO 17, SEQ ID NO 19 et SEQ ID NO 21 ou parmi les couples d'amorces constitués par une amorce répondant à la séquence nucléotidique SEQ ID NO 10 et une amorce répondant à l'une quelconque des séquences nucléotidiques SEQ ID NO 13, SEQ ID NO 15, SEQ ID NO 19 et SEQ ID NO 21.

18. Réactif pour détecter sélectivement *Streptococcus pneumoniae* dans un échantillon biologique, caractérisé en ce qu'il comprend au moins une sonde selon l'une quelconque des revendications 3 à 10.

19. Réactif selon la revendication 18, caractérisé en ce qu'il comprend une sonde selon la revendication 11.

20. Réactif selon la revendication 19, caractérisé en ce qu'il comprend une sonde selon la revendication 12.

21. Réactif selon la revendication 18, caractérisé en ce que la sonde est en milieu liquide ou fixée sur un support solide directement ou indirectement.

22. Réactif selon la revendication 21, caractérisé en ce que ledit support est choisi parmi les polystyrènes, les copolymères styrène-butadiène, les copolymères styrène-butadiène en mélange avec des polystyrènes, des polypropylènes, des polycarbonates, des copolymères polystyrène-acrylonitrile, des copolymères styrène-méthylméthacrylate de méthyle, parmi les fibres synthétiques et naturelles, parmi les polysaccharides et les dérivés de la cellulose.

23. Réactif selon la revendication 18, caractérisé en ce qu'il comprend en outre au moins une amorce selon la revendication 14 ou 15.

24. Réactif selon la revendication 23, caractérisé en ce qu'il comprend un couple d'amorces selon la revendication 16 ou 17.

25. Procédé de détection sélective de *Streptococcus pneumoniae* dans un échantillon biologique caractérisé en ce qu'on expose l'ADN génomique des bactéries contenues dans ledit échantillon, sous forme de fragments monocaténaires, à une sonde selon l'une quelconque des revendications 3 à 10 ou selon les revendications 12 et 13, et on détecte les zones d'hybridation avec ladite sonde.

26. Procédé selon la revendication 25, caractérisé en ce qu'on expose l'ADN génomique des bactéries, à une sonde de détection selon la revendication 13.

27. Procédé selon la revendication 26, caractérisé en ce qu'avant d'exposer l'ADN génomique des bactéries à ladite sonde de détection, on l'expose à une sonde de capture selon la revendication 11.

28. Procédé selon lune quelconque des revendication 25 à 27, caractérisé en ce que avant d'exposer lesdits fragments à ladite sonde, on réalise une amplification de l'ADN génomique de *Streptococcus pneumoniae,* en présence d'un système enzymatique adapté et au moins une amorce selon la revendication 14 ou 15 ou un couple d'amorces selon la revendication 16 ou 17.

## Patentansprüche

1. Einzelsträngiges genomisches DNA-Fragment aus *Streptococcus pneumoniae,* dadurch gekennzeichnet, daß es zumindest eine Nukleotidsequenz enthält, die eine Homologie von zumindest 70 % zu zumindest einer Nukleotidsequenz aufweist, die ausgewählt ist aus den Nukleotidsequenzen SEQ ID NO 2, SEQ ID NO 3, SEQ ID NO 4 und deren jeweiligen komplementären Sequenzen.

2. Fragment nach Anspruch 1, dadurch gekennzeichnet, daß die Nukleotidsequenz des genannten Fragments eine Homologie von zumindest 85 % zu zumindest einer Nukleotidsequenz aufweist, die ausgewählt ist aus den Nukleotidsequenzen SEQ ID NO 2, SEQ ID NO 3, SEQ ID NO 4 und deren jeweiligen komplementären Sequenzen.

3. Sonde, die dazu geeignet ist, spezifisch mit der genomischen DNA von *Streptococcus pneumoniae* zu hybridisieren, dadurch gekennzeichnet, daß sie eine Nukleotidsequenz enthält, die eine Homologie von zumindest 70 % zu zumindest einem Teil einer Konsensussequenz der genomischen DNA von *Streptococcus pneumoniae,* die ausgewählt ist aus den Nukleotidsequenzen SEQ ID NO 2, SEQ ID NO 3, SEQ ID NO 4 und deren jeweiligen komplementären Sequenzen, aufweist und die zugleich nur mit der genomischen DNA von *Streptococcus pneumoniae* hybridisiert.

4. Sonde nach Anspruch 3, dadurch gekennzeichnet, daß die Nukleotidsequenz der genannten Sonde eine Homologie von zumindest 85 % zu zumindest einem Teil der genannten Konsensussequenz aufweist.

5. Sonde nach Anspruch 3 oder 4, dadurch gekennzeichnet, daß die Nukleotidsequenz der genannten Sonde zumindest zwölf Nukleotide aufweist.

6. Sonde nach einem der Ansprüche 3 bis 5, dadurch gekennzeichnet, daß sie die Nukleotidsequenz SEQ ID NO 3 aufweist.

7. Sonde nach Anspruch 6, dadurch gekennzeichnet, daß die genannte Nukleotidsequenz SEQ ID NO 3 an ihrem 5'-Ende von einer Nukleotidsequenz SEQ ID NO 2 flankiert wird.

8. Sonde nach Anspruch 6, dadurch gekennzeichnet, daß die genannte Nukleotidsequenz SEQ ID NO 3 an ihrem 3'-Ende von einer Nukleotidsequenz SEQ ID NO 4 flankiert wird.

9. Sonde nach Anspruch 6, dadurch gekennzeichnet, daß die Nukleotidsequenz SEQ ID NO 3 viermal aneinandergrenzend wiederholt vorliegt.

10. Sonde nach Anspruch 3, dadurch gekennzeichnet, daß sie aus einer Nukleotidsequenz aufgebaut ist, die ausgewählt ist aus den Sequenzen SEQ ID NO 5, SEQ ID NO 6 und SEQ ID NO 7.

11. Sonde zum Fischen denaturierter DNA, dadurch gekennzeichnet, daß sie ausgewählt ist aus den Sonden nach einem der Ansprüche 3 bis 10.

12. Sonde nach einem der Ansprüche 1 bis 10, dadurch gekennzeichnet, daß sie mittels eines Markers markiert ist, der ausgewählt ist aus radioaktiven Isotopen, Enzymen, die ausgewählt sind aus Peroxydase und Alkalischer Phosphatase und solchen Enzymen, die dazu geegnet sind, ein chromogenes, fluorigenes oder lumineszierendes Substrat zu hydrolysieren, aus chromophoren chemischen Verbindungen, chromogenen, fluorigenen oder lumineszierenden Verbindungen, Basenanalogen von Nukleotiden und Biotin.

13. Sonde zum Nachweisen, dadurch gekennzeichnet, daß sie ausgewählt ist aus den Sonden nach Anspruch 12.

14. Spezifischer Primer zur Amplifikation der genomischen DNA von *Streptococcus pneumoniae* durch Polymerase-Kettenreaktion, dadurch gekennzeichnet, daß er eine Nukleotidsequenz enthält, die eine Homologie von zumindest 70 % zu zumindest einem Teil einer Konsensussequenz der genomischen DNA von *Streptococcus pneumoniae* aufweist, die ausgewählt ist aus den Nukleotidsequenzen SEQ ID NO 2, SEQ ID NO 3, SEQ ID NO 4 und deren jeweiligen komplementären Sequenzen, und die zugleich nur mit der genomischen DNA von *Streptococcus pneumoniae* hybridisiert.

15. Primer nach Anspruch 14, dadurch gekennzeichnet, daß er ausgewählt ist aus den Primern, die jeweils den Nukleotidsequenzen SEQ ID NO 8 bis SEQ ID NO 21 entsprechen.

16. Primerpaar, dadurch gekennzeichnet, daß es zumindest einen Primer aufweist, der ausgewählt ist aus den Primern nach Anspruch 15.

17. Primerpaar nach Anspruch 16, dadurch gekennzeichnet, daß es ausgewählt ist aus den Primerpaaren, die aus einem Primer, der der Nukleotidsequenz SEQ ID NO 8 entspricht, und einer Sonde bestehen, die einer beliebigen der Nukleotidsequenzen SEQ ID NO 11, SEQ ID NO 13, SEQ ID NO 15, SEQ ID NO 17, SEQ ID NO 19 und SEQ ID NO 21 entspricht, oder aus den Primerpaaren ausgewählt ist, die aus einem Primer, der der Nukleotidsequenz SEQ ID NO 10 entspricht, und einem Primer bestehen, der einer beliebigen der Nukleotidsequenzen SEQ ID NO 13, SEQ ID NO 15, SEQ ID NO 19 und SEQ ID NO 21 entspricht.

18. Reaktives Agens zum selektiven Nachweis von *Streptococcus pneumoniae* in einer biologischen Probe, dadurch gekennzeichnet, daß es zumindest eine Sonde nach einem der Ansprüche 3 bis 10 aufweist.

19. Reaktives Agens nach Anspruch 18, dadurch gekennzeichnet, daß es eine Sonde nach Anspruch 11 aufweist.

20. Reaktives Agens nach Anspruch 19, dadurch gekennzeichnet, daß es eine Sonde nach Anspruch 12 aufweist.

21. Reaktives Agens nach Anspruch 18, dadurch gekennzeichnet, daß sich die Sonde in flüssigem Milieu befindet oder direkt oder indirekt an einen festen Träger fixiert ist.

22. Reaktives Agens nach Anspruch 21, dadurch gekennzeichnet, daß der Träger ausgewählt ist aus Polystyrolen, Styrol-Butadien-Copolymeren, mit Polystyrolen vermischten Styrol-Butadien-Copolymeren, Polypropylenen, Polycarbonaten, Polystyrol-Acrylnitril-Copolymeren, Styrol-Methylmethacrylatmethylester-Copolymeren, Polysacchariden und Cellulosederivaten.

23. Reaktives Agens nach Anspruch 18, dadurch gekennzeichnet, daß er ferner zumindest einen Primer nach Anspruch 14 oder 15 aufweist.

24. Reaktives Agens nach Anspruch 23, dadurch gekennzeichnet, daß es ein Primerpaar nach Anspruch 16 oder 17 aufweist.

25. Verfahren zum selektiven Nachweis von *Streptococcus pneumoniae* in einer biologischen Probe, dadurch gekennzeichnet, daß die in der genannten Probe enthaltene genomische DNA der Bakterien in Form von einzelsträngigen Fragmenten einer Sonde nach einem der Ansprüche 3 bis 10 oder nach den Ansprüchen 12 und 13 ausgesetzt wird, und daß die mit der Probe hybridisierenden Abschnitte nachgewiesen werden.

26. Verfahren nach Anspruch 25, dadurch gekennzeichnet, daß die genomische DNA der Bakterien einer Sonde zum Nachweisen nach Anspruch 13 ausgesetzt wird.

27. Verfahren nach Anspruch 26, dadurch gekennzeichnet, daß die genomische DNA der Bakterien einer Sonde zum Fischen nach Anspruch 11 ausgesetzt wird, bevor sie der genannten Sonde zum Nachweisen ausgesetzt wird.

28. Verfahren nach einem der Ansprüche 25 bis 27, dadurch gekennzeichnet, daß, bevor die Fragmente der genannten Sonde ausgesetzt werden, eine Amplifikation der genomischen DNA von *Streptococcus pneumoniae* in Anwesenheit eines daran angepaßten enzymatischen Systems und in Anwesenheit von zumindest einem Primer nach Anspruch 14 oder 15 oder einem Primerpaar nach Anspruch 16 oder 17 durchgeführt wird.

## Claims

1. A single-stranded fragment of the genomic DNA of *Streptococcus pneumoniae,* which comprises at least one nucleotide sequence having at least 70% homology with at least one nucleotide sequence selected from the nucleotide sequences SEQ ID NO 2, SEQ ID NO 3, SEQ ID NO 4, and their respective complementary sequences.

2. A fragment as claimed in claim 1, wherein the nucleotide sequence of said fragment has at least 85% homology with at least one nucleotide sequence selected from the nucleotide sequences SEQ ID NO 2, SEQ ID NO 3, SEQ ID NO 4, and their respective complementary sequences.

3. A probe capable of specifically hybridizing with the genomic DNA of *Streptococcus pneumoniae,* which comprises a nucleotide sequence, both, at once, having at least 70% homology with at least a portion of a consensus sequence of the genomic DNA of *Streptococcus pneumoniae,* this consensus sequence being selected from the nucleotide sequences SEQ ID NO 2, SEQ ID NO 3, SEQ ID NO 4, and their respective complementary sequences, and only hybridizing with the genomic DNA of *Streptococcus pneumoniae.*

4. The probe as claimed in claim 3, wherein the nucleotide sequence which said probe comprises, as at least 85% homology with at least a portion of said consensus sequence.

5. The probe as claimed in claim 3 or 4, wherein the nucleotide sequence which said probe comprises, contains at least 12 nucleotides.

6. The probe as claimed in any one of claims 3 to 5, which comprises the nucleotide sequence SEQ ID NO 3.

7. The probe as claimed in claim 6, wherein said nucleotide sequence SEQ ID NO 3 is flanked at its 5' end by a nucleotide sequence SEQ ID NO 2.

8. The probe as claimed in claim 6, wherein said nucleotide sequence SEQ ID NO 3 is flanked at its 3' end by a nucleotide sequence SEQ ID NO 4.

9. The probe as claimed in claim 6, wherein the nucleotide sequence SEQ ID NO 3 is repeated four times contiguously.

10. The probe as claimed in claim 3, which consists of a nucleotide sequence selected from the sequences SEQ ID NO 5, SEQ ID NO 6 and SEQ ID NO 7.

11. A capture probe for denatured DNA, which is selected from the probes as claimed in any one of claims 3 to 10.

12. The probe as claimed in any one of claims 1 to 10, which is labeled by means of a marker selected from radioactive isotopes, from enzymes selected from peroxidase and alkaline phosphatase and those capable of hydrolyzing a chromogenic, fluorigenic or luminescent substrate, from chromophoric chemical compounds, from chromogenic, fluorigenic or luminescent compounds, from nucleotide base analogs and from biotin.

13. A detection probe, which is selected from the probes as claimed in claim 12.

14. A specific primer for the amplification, by polymerization, of the genomic DNA of *Strepsococcus pneumoniae,* which has a nucleotide sequence, both, at once, having at least 70% homology with at least a portion of a consensus sequence of the genomic DNA of *Streptococcus pneumoniae*, this consensus sequence being selected from the nucleotide sequences SEQ ID NO 2, SEQ ID NO 3, SEQ ID NO 4, and their respective complementary sequences, and only hybridizing with the genomic DNA of *Streptococcus pneumoniae.*

15. The primer as claimed in claim 14, which is selected from the primers of the nucleotide sequences SEQ ID NO 8 to SEQ ID NO 21.

16. A pair of primers which comprises at least one primer selected from the primers as claimed in claim 15.

17. The pair of primers as claimed in claim 16, which is selected from the pairs of primers consisting of a primer of the nucleotide sequence SEQ ID NO 8 and a primer of any one of the nucleotide sequences SEQ ID NO 11, SEQ ID NO 13, SEQ ID NO 15, SEQ ID NO 17, SEQ ID NO 19 and SEQ ID NO 21, or from the pairs of primers consisting of a primer of the nucleotide sequence SEQ ID NO 10 and a primer of any one of the nucleotide sequences SEQ ID NO 13, SEQ ID NO 15, SEQ ID NO 19 and SEQ ID NO 21.

18. A reagent for selectively detecting *Streptococcus pneumoniae* in a biological sample, which comprises at least one probe as claimed in any one of claims 3 to 10.

19. The reagent as claimed in claim 18, which comprises one probe as claimed in claim 11.

20. The reagent as claimed in claim 19, which comprises a probe as claimed in claim 12.

21. The reagent as claimed in claim 18, wherein the probe is in liquid medium or directly or indirectly fixed on a solid support.

22. The reagent as claimed in claim 21, wherein said support is selected from polystyrenes, styrene/butadiene copolymers, styrene/butadiene copolymers mixed with polystyrenes, polypropylenes, polycarbonates, polystyrene/acrylonitrile copolymers, styrene-methyl methacrylate copolymers, from synthetic nylon and natural fibers, from polysaccharides and cellulose derivatives.

23. The reagent as claimed in claim 18, which additionally comprises at least one primer as claimed in claim 14 or 15.

24. The reagent as claimed in claim 23, which comprises a pair of primers as claimed in claim 16 or 17.

25. Method for the selective detection of *Streptococcus pneumoniae* in a biological sample, wherein the genomic DNA of the bacteria contained in said sample, in the form of single-stranded fragments, is exposed to a probe as claimed in any one of claims 3 to 10 or as claimed in claims 12 and 13, and the regions of hybridization with said probe are detected.

26. The method as claimed in claim 25, wherein the genomic DNA of bacteria is exposed to a detection probe as claimed in claim 13.

27. The method as claimed in claim 26, wherein, before exposing the genomic DNA of bacteria to said detection probe, it is exposed to a capture probe as claimed in claim 11.

28. The method as claimed in any one of claims 25 to 27, wherein, before exposing said fragments to said probe, the genomic DNA of *Streptococcus pneumoniae* is amplified in the presence of an appropriate enzymatic system and at least one primer as claimed in claim 14 or 15 or a pair of primers as claimed in claim 16 or 17.
